# EUROPEAN PATENT APPLICATION

(11) **EP 3 785 692 A1**
(43) Date of publication of application: **03.03.2021**
(21) Application number: 19194736.5
(22) Date of filing: 30.08.2019
(51) Int. Cl.: A61K 6/00, C08L 33/10

(54) **DENTAL COMPOSITION**

(71) Applicant: DENTSPLY SIRONA Inc., York, PA 17401 (US); DENTSPLY DETREY GmbH, 78467 Konstanz (DE)
(72) Inventor: Klee, Prof. Dr. Joachim E., 78315 Radolfzell (DE); Szillat, Dr. Florian, 78462 Konstanz (DE); Kirschner, Julie, 67650 Dambach-la-Ville (FR); Lalevée, Prof. Dr. Jacques, 68200 Mulhouse (FR)
(74) Representative: Dietz, Mirko

(57) **Abstract**

The present invention is related to a dental composition comprising at least one polymerizable monomer having at least one ethylenically unsaturated group; and a radical initiator system comprising a photosensitizer having an absorption maximum ranging from 400 nm to 800 nm; and at least one CH- acidic compound as coinitiator.

## Description

### Field of the Invention

The present invention relates to a dental composition comprising at least one polymerizable monomer having at least one ethylenically unsaturated group and a radical initiator system, which comprises a photosensitizer having an absorption maximum ranging from 400 nm to 800 nm and at least one CH- acidic compound as coinitiator.

### Background of the Invention

The restoration of teeth commonly involves a photocurable dental composition containing free radically polymerizable resins. Photocuring of a dental composition involves a photoinitiator system generating free radicals upon exposure to visible light. Conventionally, photosensitizers such as camphorquinone (CQ) optionally in combination with a tertiary aromatic amine are frequently used as photoinitiator systems.

However, the presence of amines in (meth)acrylate containing compositions can cause yellowing in the resulting photocured composition. Another disadvantage of the tertiary aromatic amine is their discoloration tendency and their sensitivity to strong acidic media. Furthermore, aromatic tertiary amines react with oxygen radicals. Thus, while beneficial on the one hand that its use avoids a sticky surface, on the other hand more aromatic amine is needed for initiation than the stoichiometric requirement with the respective photosensitizer such as CQ. Tertiary amines for use as coinitiators in dental compositions may leach out of the cured composition which may give rise to toxicological concern. Especially, the toxicological concerns about tertiary aromatic amines requires the development of new coinitiators for photosensitizers such as CQ in dental materials.

### Objective of the present Invention

In view of the prior art, it was thus an object of the present invention to provide a new dental composition comprising an initiator system, which shall not exhibit the aforementioned shortcomings of the known prior art dental compositions.

In particular, it was an object of the present invention to provide a dental composition comprising an initiator system, which shall be at least substantially free, preferably completely free, of amines, especially of tertiary amines, more especially of tertiary aromatic amines.

What is needed therefore is a way to provide new coinitiators for common photosensitizers, such as camphorquinone, in dental compositions, wherein the coinitiators are free of amines.

Furthermore, it was an object of the present invention to provide a dental composition comprising a radical initiator system containing a combination of a photosensitizer and at least one coinitiator being a CH-acidic compound, wherein such an inventive dental composition can reduce, or ideally avoid or eliminate, the leaching problems and the toxicological concerns of the commonly uses coinitiators based on tertiary aromatic amines, while improving the required biocompatibility simultaneously.

### Summary of the Invention

These objects and also further objects which are not stated explicitly but are immediately derivable or discernible from the connections discussed herein by way of introduction are achieved by a dental composition having all features of claim 1. Appropriate modifications of the dental composition are protected in dependent claims 2 to 15.

The present invention accordingly provides a dental composition comprising
a) at least one polymerizable monomer having at least one ethylenically unsaturated group;
b) a radical initiator system comprising
   i. a photosensitizer having an absorption maximum ranging from 400 nm to 800 nm; and
   ii. at least one CH- acidic compound as coinitiator.

It is thus possible in an unforeseeable manner to provide a new dental composition comprising an initiator system, which does not exhibit the aforementioned shortcomings of the known prior art dental compositions.

In particular, the present invention provides a dental composition comprising an initiator system, which is at least substantially free, preferably completely free, of amines, especially of tertiary amines, more especially of tertiary aromatic amines.

Thereby, the present invention provides new coinitiators for common photosensitizers, such as camphorquinone, in dental compositions, wherein the coinitiators are free of amines.

Furthermore, a dental composition is provided, which comprises a radical initiator system containing a combination of a photosensitizer and at least one coinitiator being a CH-acidic compound, wherein such an inventive dental composition can reduce, or ideally avoid or eliminate, the leaching problems and the toxicological concerns of the commonly uses coinitiators based on tertiary aromatic amines, while improving the required biocompatibility simultaneously.

The dental composition comprising at least one CH-acidic compound as coinitiator provides improved polymerization efficiency including a good conversion without posing leaching problems of aromatic amines. Such a coinitiator being a CH-acidic compound of the present invention is stable to strong acidic media and thereby suitable for dental application such as adhesives and self-etching preparations. Such coinitiators of the present invention when being incorporated into such a dental composition have low tendency towards discoloration while exhibiting good storage stability at the same time.

The at least one coinitiator being a CH-acidic compound acts by initiating polymerization of one or more polymerizable monomers having at least one ethylenically unsaturated groups. The coinitiators of the present invention provide biocompatibility with reduced release of components when incorporated into such dental compositions.

### Brief Description of the Figures

For a more complete understanding of the present invention, reference is made to the following Detailed Description of the Invention considered in conjunction with the accompanying figures, in which:

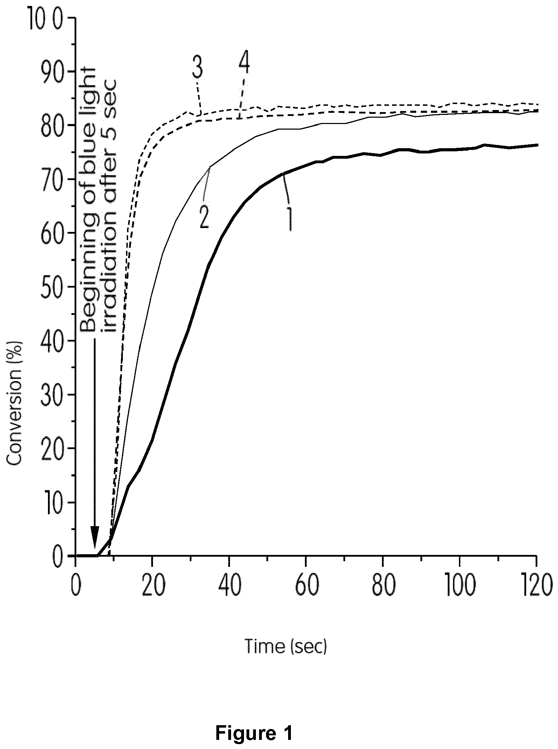

**FIG. 1** depicts photopolymerization profiles of methacrylate functions (TPH3) in presence of (1) CQ (0.5% wt) or (2) CQ/lactone (HD-8) (0.5/3% w/w) or (4) CQ/ DMABE (0.5/1% w/w) or (3) CQ/lactone (HD-8)/Speedcure 938 (0.5/3/1% w/w) under exposure to Smartlite Focus (300 mW.cm-2); sample thickness 1.4mm; under air.

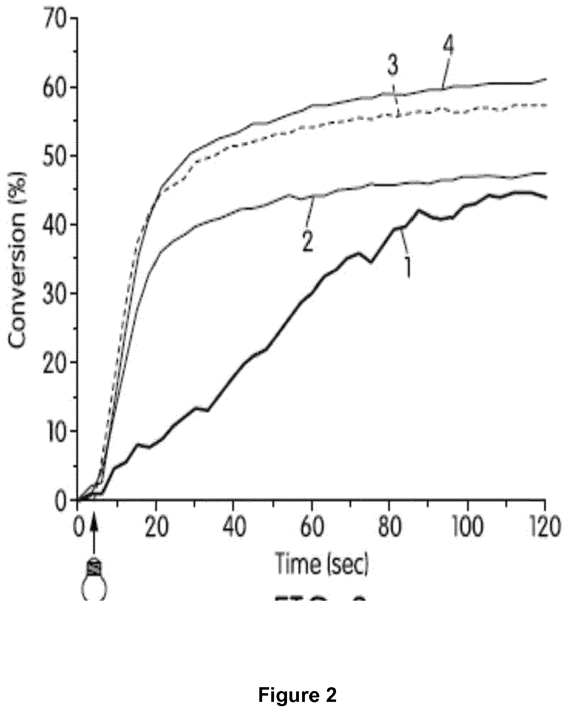

**FIG. 2** depicts photopolymerization profiles of C=C double bonds (PrimeBond Active) in presence of (1) CQ (1.5% w/w) or (2) CQ/Speedcure 938 (1.5/0.75 % w/w) or (3) CQ/acetylbutyrolactone (HD-8)/iodonium sulfonate (1.5/1.2/0.75 % w/w) or (4) CQ/acetylbutyrolactone (HD-8)/Speedcure938 (1.5/1.2/0.75 % w/w) or under exposure to SmartLite Focus (300mW.cm-2); sample thickness 17µm; under air.

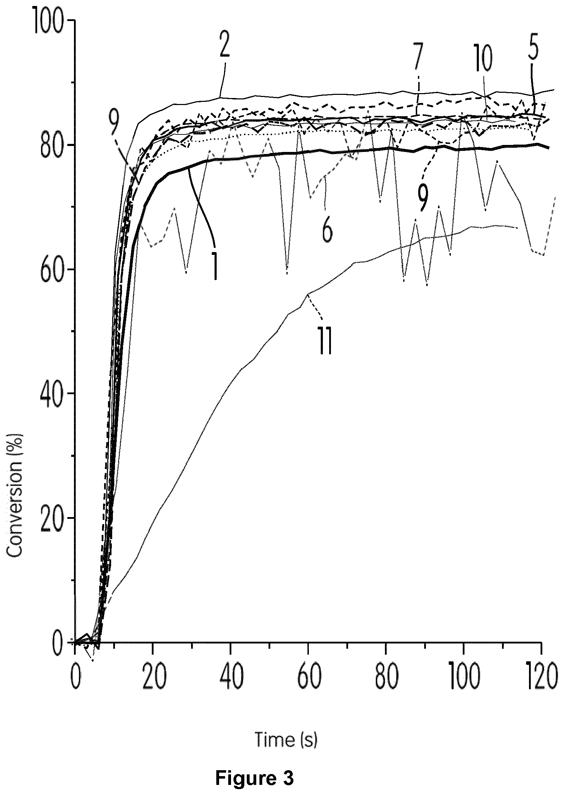

**FIG. 3** depicts photopolymerization profiles of methacrylate functions (TPH3) in presence of camphorquinone as photoinitiator (under air; thickness = 1.4 mm; SmartLite Focus 300 mW.cm-2): (1) CQ/HD-1/SC938 (0.5/3/1 % w/w) (2) CQ/HD-2/SC938 (0.5/3/1 % w/w) (3) CQ/HD-3/SC938 (0.5/3/1 % w/w) (4) CQ/HD-4/SC938 (0.5/3/1 % w/w) (5) CQ/HD-5/SC938 (0.5/3/1 % w/w) (6) CQ/HD-6/SC938 (0.5/3/1 % w/w) (7) CQ/HD-7/SC938 (0.5/3/1 % w/w) (8) CQ/HD-8/SC938 (0.5/3/1 % w/w) (9) CQ/HD-10/SC938 (0.5/3/1 % w/w) (10) CQ/DMABE (0.5/0.5 % w/w) (11) CQ (0.5% w/w). The irradiation starts for t = 5 s.

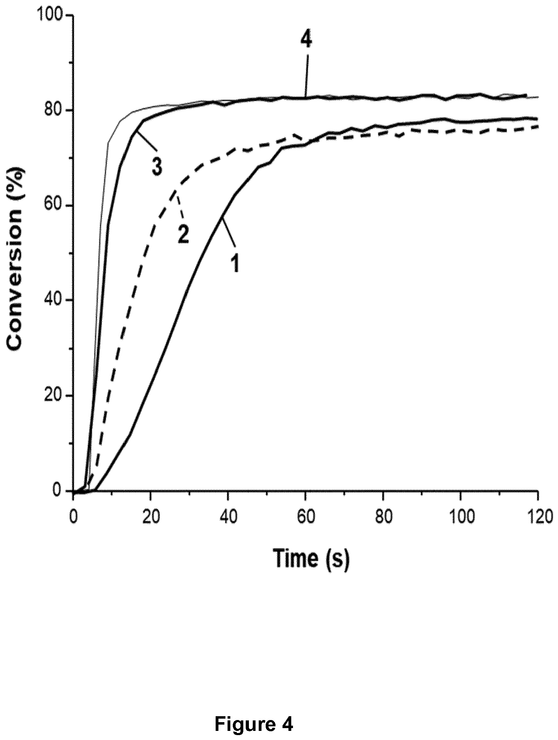

**FIG. 4** represents photopolymerization profiles of methacrylate functions (TPH3) in presence of camphorquinone as photoinitiator (under air; thickness = 1.4 mm; SmartLite Focus 300 mW.cm-2): (1) CQ/TPH3 (0.5/100 % w/w) (2) CQ/HD-9/TPH3 (0.5/5/95 % w/w) (3) CQ/HD-9/SC938/TPH3 (0.5/5/1/100 % w/w) (4) CQ/DMABE/TPH3 (0.5/0.6/100 % w/w) (5) CQ/DMABE/HD-9/TPH3 (0.5/0.6/5/95 % w/w). The irradiation starts for t = 5 s.

### Detailed Description of the Invention

The term "alkyl", unless otherwise specified, refers to a monoradical branched or unbranched saturated hydrocarbon chain having from 1 to 18 carbon atoms. This term can be exemplified by groups such as methyl, ethyl, n-propyl, iso-propyl, n-butyl, iso-butyl, sec-butyl, t-butyl, n-pentyl, isopentyl, neopentyl, n-hexyl, n-decyl, dodecyl, tetradecyl, and the like. Alkyl groups may be substituted further with one or more substituents selected from alkenyl, alkoxy, and hydroxyl.

The term "alkylene", unless otherwise specified refers to a linear saturated divalent hydrocarbon radical of one to four carbon atoms or a branched saturated divalent hydrocarbon radical of three to four carbon atoms, e.g., methylene, ethylene, 2,2-dimethylethylene, propylene, 2-methylpropylene, butylene and the like, preferably methylene, ethylene, or propylene.

The term "aryl" refers to C6-C10-membered aromatic, heterocyclic, fused aromatic, fused heterocyclic, biaromatic, or bihetereocyclic ring systems. Broadly defined, "aryl", as used herein, includes 5-, 6-, 7-, 8-, 9-, and 10-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine, pyrimidine, and the like. Those "aryl" groups having heteroatoms in the ring structure may also be referred to as "heteroaryl" or "heterocycles" or "heteroaromatics". The aromatic ring can be substituted at one or more ring positions with one or more substituents including, but not limited to, halogen, azide, alkyl, aralkyl, alkenyl, alkynyl, cycloalkyl, hydroxyl, alkoxyl, amino (or quaternized amino), nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties,-CF3, --CN, and combinations thereof.

The term "cycloalkyl" refers to monocyclic or polycyclic cycloalkyl radical. Examples of monocyclic acycloakyl include cyclopropyl, cyclobutyl, cyclopentyl, cyclohexyl, cycloheptyl, and cyclooctyl. Examples of polycyclic cycloalkyl radical include, for example admantyl, norbornyl, decalinyl, 7,7-dimethyl-bicyclo[2.2.1]heptanyl, tricyclo[5.2.1.02,6]decyl and the like. Unless otherwise stated specifically in the specification, the term "cycloalkyl" is meant to include monocyclic or polycyclic cycloalkyl radical that are optionally substituted by one or more substituents selected from alkyl, halo, oxo or alkylene chain.

The term "(meth)acrylate" in the context of the present disclosure is meant to refer to the acrylate as well as to the corresponding methacrylate.

The term "(meth)acrylamide" in the context of the present disclosure is meant to include acrylamide and methacrylamide.

The term "polymerizable monomer" in the context of the present disclosure means any monomer capable of radical polymerization. The polymerizable monomer includes at least one ethylenically unsaturated groups. The at least one ethylenically unsaturated groups include vinyl, allyl, acryl, methacryl, and styryl.

The term "a polymerizable monomer having at least one ethylenically unsaturated group" and "ethylenically unsaturated monomers" may be used interchangeably.

The term "radical initiator system" in the context of the present disclosure means any system comprising a sensitizer and at least one coinitiator forming free radicals when activate by thermal or light and/or ambient redox conditions, whereby polymerization of polymerizable monomer is initiated.

The term "coinitiator" in the context of the present disclosure means a compound that does not essentially absorb when exposed with UV radiation or visible light but forms free radicals together with the sensitizers used according to the present disclosure.

The term "sensitizer" in the context of the present disclosure which can absorb radiation of a wavelength in the range of 400 to 800 nm, when it is exposed but which cannot by itself, i.e. without the addition of coinitiators, form free radicals. Sensitizers used in the present disclosure have to be capable of interacting with the coinitiators used in the present disclosure.

The present disclosure relates to a dental composition. The dental composition is selected from a dental adhesive, a dental composite, a root canal filling composition, a dental sealant and a dental cement.

### The polymerizable monomers

The dental composition of the present disclosure comprises one or more polymerizable monomers having at least one ethylenically unsaturated group.

Polymerizable monomers may be acrylates, methacrylates, ethylenically unsaturated compounds, carboxyl group-containing unsaturated monomers, C2-8 hydroxyl alkyl esters of (meth)acrylic acid, C1-24 alkyl esters or cycloalkyl esters of (meth)acrylic acid, C2-18 alkoxyalkyl esters of (meth)acrylic acid, olefins or diene compounds, monoesters/diesters, monoethers, adducts, vinyl monomer, styryl monomer, TPH resin, SDR Resin, PBA resins and/or BPA-free resins.

Examples of specific acrylate monomer include, but are not limited to, methyl acrylate, ethyl acrylate, propyl acrylate, isopropyl acrylate, 2-hydroxyethyl acrylate, hydroxypropyl acrylate, tetrahydrofurfuryl acrylate, glycidyl acrylate, glycerol mono- and di- acrylate, ethylene glycol diacrylate, polyethyleneglycol diacrylate, neopentyl glycol diacrylate, trimethylolpropane triacrylate, mono-, di-, tri-acrylate, mono-, di-, tri-, and tetra-acrylates of pentaerythritol and dipentaerythritol, 1,3-butanediol diacrylate, 1 ,4-butanedioldiacrylate, 1 ,6- hexanediol diacrylate, 2,2'- bis[3(4-phenoxy)-2-hydroxypropane-1 -acrylate]propane, 2,2'-bis(4- acryloxyphenyl)propane, 2,2'-bis[4(2-hydroxy-3-acryloxy-phenyl)]propane, 2,2'-bis(4-acryloxyethoxyphenyl)propane, 2,2'-bis(4-acryloxypropoxyphenyl)propane,2,2'- bis(4-acryloxydiethoxyphenyl)propane, 2'-bis[3(4-phenoxy)-2-hydroxypropane-1 -acrylate]propane, and dipentaerthritol pentaacrylate esters.

Examples of specific conventional methacrylate monomer include, but are not limited to, methyl methacrylates, ethyl methacrylate, propyl methacrylate, iso-propyl methacrylate, tetrahydrofurfuryl methacrylate, glycidyl methacrylate, the diglycidyl methacrylate of bis-phenol A (2,2-Bis[4-(2-hydroxy-3- methacryloxypropoxy)phenyl]propane) (BisGMA), 4,4,6,16 (or 4,6,6,16)-tetramethyl-10,15-dioxo-11,14-dioxa-2,9-diazaheptadec-16-enoicacid 2-[(2-methyl-1-oxo-2-propen-1-yl)oxy]ethyl ester (CAS no. 72869-86-4)(UDMA), glycerol mono- and di-methacrylate, ethyleneglycol dimethacrylate, polyethyleneglycol dimethacrylate, triethylene glycol dimethacrylate (TEGDMA), neopentylglycol dimethacrylate, trimethylol propane trimethacrylate, mono-, di-, tri-, and tetra-methacrylates of pentaerythritol and dipentaerythritol, 1 ,3-butanediol dimethacrylate, 1 ,4-butanediol dimethacrylate, Bis[2- (methacryloyloxy)ethyl]phosphate (BisMEP),1 ,6-hexanediol dimethacrylate, 2-2'-bis(4- methacryloxyphenyl)propane, 2,2'-bis[4(2-hydroxy-3-methacryloxy-phenyl)]propane, 2,2'-bis(4-methacryloxyethoxyphenyl)propane, 2,2'-bis(4-methacryloxypropoxyphenyl)propane, , 2,2'-bis(4-methacryloxydiethoxyphenyl)propane , 2,2'-bis[3(4-phenoxy)-2-hydroxypropane-1 - methacrylate]propane, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1 -chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, and methylene-bis-1-chloromethyl-2-methacryloxyethyl-4-cyclohexyl carbamate.

Examples of ethylenically unsaturated compounds include, but are not limited to, acrylic acid esters, methacrylic acid esters, hydroxy-functional acrylic acid esters, hydroxy-functional methacrylic acid esters, halogen, and hydroxy containing methacrylic acid esters and combinations thereof. Such free radically polymerizable compound include n-, sec-, or t-butyl methacrylate, hexyl methacrylate, 2-ethylhexyl methacrylate, octylmethacrylate, decyl methacrylate, lauryl methacrylate, cyclohexyl methacrylate, stearyl(meth)acrylate, allyl(meth)acrylate, glycerol tri(meth)acrylate, diethyleneglycol di(meth)acrylate, triethyleneglycol di(meth)acrylate, 1,3-propanediol di(meth)acrylate, 1,2,4-butanetriol tri(meth)acrylate, 1,4-cyclohexanediol di(meth)acrylate, pentaerythritol tetra(meth)acrylate, sorbitol hex(meth)acrylate, bis[1-(2-acryloxy)]-p-ethoxyphenyldimethylmethane, bis[1-(3-acryloxy-2-hydroxy)]-p-propoxyphenyldimethylmethane, ethoxylated bisphenol A di(meth)acrylate, and trishydroxyethyl-isocyanurate tri(meth)acrylate; urethane (meth)acrylates; ((di)urethane dimethacrylate), the bis-(meth)acrylates of polyethylene glycols, and chlorine-, bromine-, fluorine-, and hydroxyl group containing monomers, for example, 3-chloro-2-hydroxylpropyl (meth)acrylate, and reaction product of Bisphenol-A-Glycidylmethacrylate (BisGMA) and a hexamethylene diisocyanate (HMDI).

Examples of carboxyl group-containing unsaturated monomers include, but are not limited to, acrylic acid, methacrylic acid, crotonic acid, itaconic acid, maleic acid, and fumaric acid. Examples of C2-8 hydroxyl alkyl esters of (meth)acrylic acid include, but are not limited to, 2-hydroxylethyl (meth)acrylate, 2-hydroxylpropyl (meth)acrylate, 3-hydroxypropyl (meth)acrylate, and hydroxybutyl (meth)acrylate. Examples of C2-18 alkoxyalkyl esters of (meth)acrylic acid include, but are not limited to, methoxybutyl methacrylate, methoxyethyl methacrylate, ethoxyethyl methacrylate, and ethoxybutyl methacrylate. Specifically, acrylate and methacrylates may be

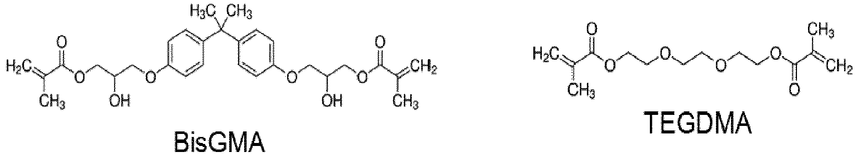

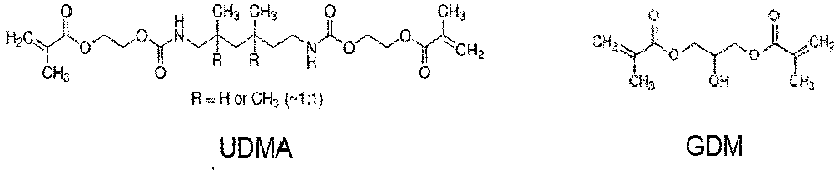

Olefins or diene compounds include, but are not limited to, ethylene, propylene, butylene, isobutene, isoprene, chloropropene, fluorine containing olefins and vinyl chloride.

Examples of monoesters may include monoesters between a polyether polyol (e.g., polyethylene glycol, polypropylene glycol or polybutylene glycol) and an unsaturated carboxylic acid (preferably methacrylic acid), monoesters or diesters between an acid anhydride group-containing unsaturated compounds (e.g., maleic anhydride or itaconic anhydride) and a glycol (e.g. ethylene glycol, 1,6-hexanediol or neopentyl glycol).

Examples of monoethers may include monoethers between a polyether polyol (e.g., polyethylene glycol, polypropylene glycol or polybutylene glycol) and a hydroxyl group containing unsaturated monomers (e.g., 2-hydroxyl methacrylate).

Examples of adducts may include, but are not limited to, adducts between an unsaturated carboxylic acid and a monoepoxy compound; adducts between glycidyl (meth)acrylates (preferably methacrylate) and a monobasic acid (e.g., acetic acid, propionic acid, p-t-butylbenzonic acid or a fatty acid).

Examples of vinyl monomers include, but are not limited, to aminopropyl vinyl ether, aminoethyl vinyl ether, N-vinyl formamide, vinylene carbonate, vinyl acetate, divinyl benzene, divinyl succinate, divinyl adipate, divinylphthalate, vinylpyridine, N-vinylpyrrolidone, vinyl carbazole, vinyldene halide, and vinyl (meth)acrylates.

Examples of styryl monomers include, but are not limited, to styrene and 2-methylstyrene. (Meth)acrylamides may be selected from the following compounds: (meth)acrylamide, methylene bis-(meth)acrylamide, diacetone (meth)acrylamide,

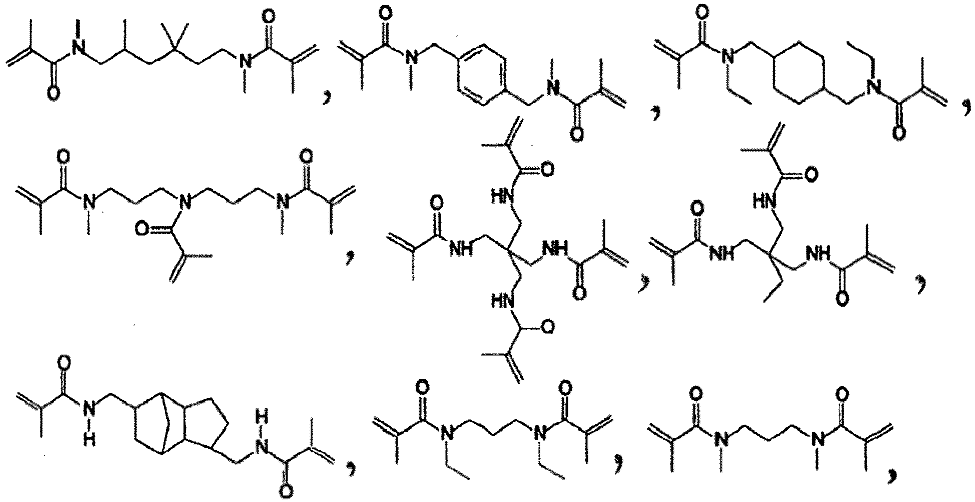

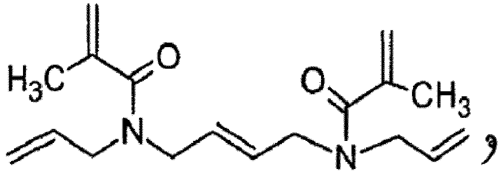

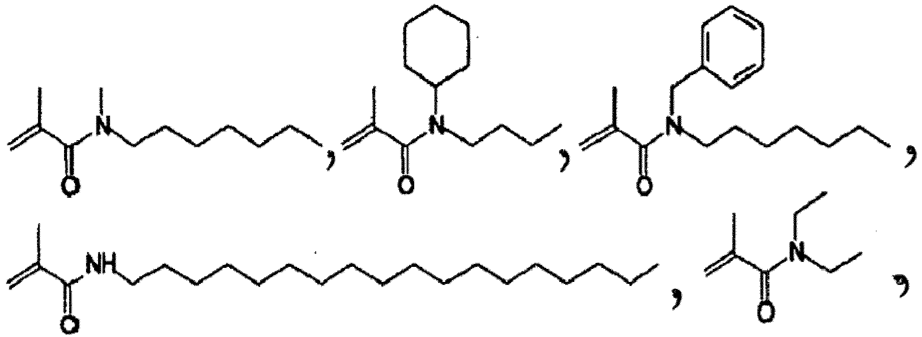

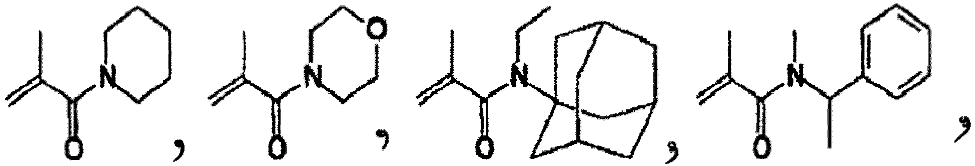

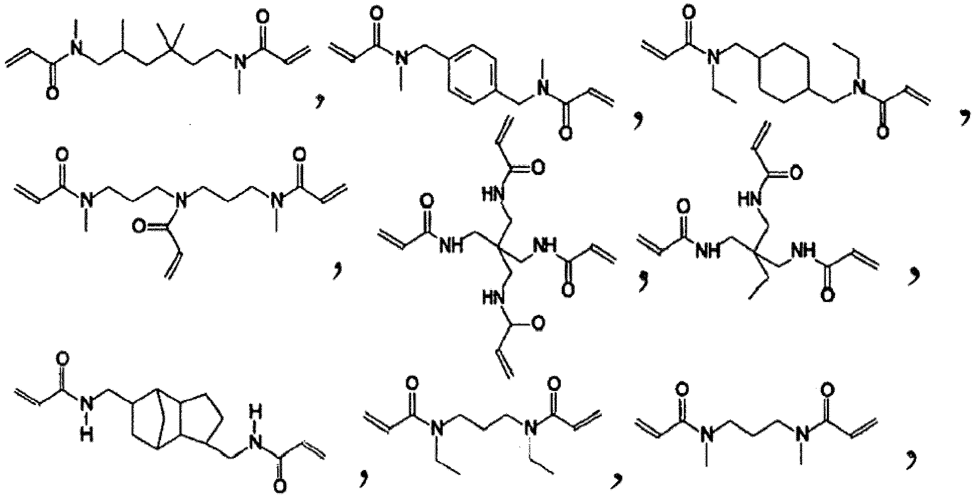

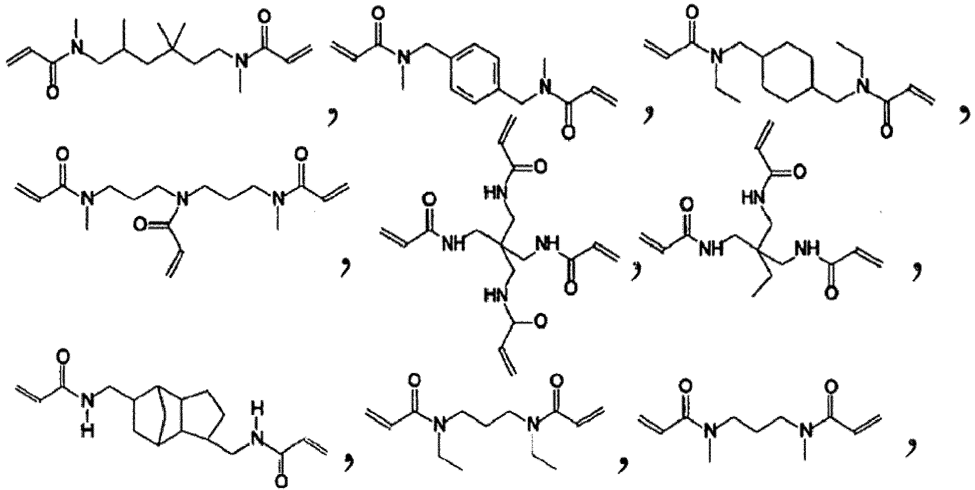

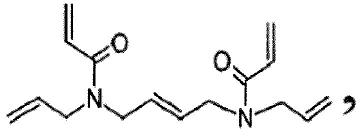

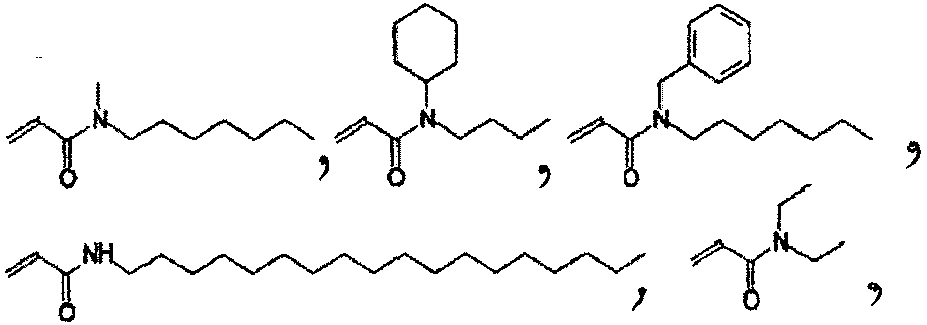

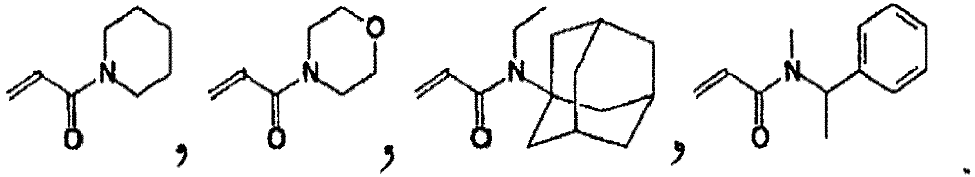

Specifically, methacrylamide may be bis-(meth)acrylamides, such as N,N'-diallyl-1,4- bisacrylamido-(2E)-but-2-en (BAABE) having the structural formula 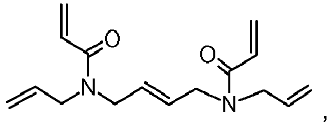 and/or
N,N'-diethyl-1,3-bisacrylamido-propan (BADEP) having the structural formula

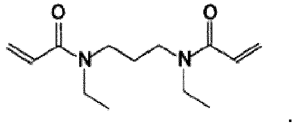

One or more polymerizable monomers having at least one ethylenically unsaturated group may also be phosphoric acid ester group containing polymerizable monomers having at least one radically polymerizable double bond.

Preferably, such phosphoric acid ester group containing polymerizable monomers have the following Formula (A): 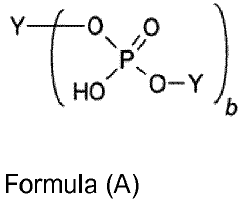 wherein
the moieties Y independent from each other represent a hydrogen atom or a moiety of the following formulae (Y*), (Y**) or (Y***): 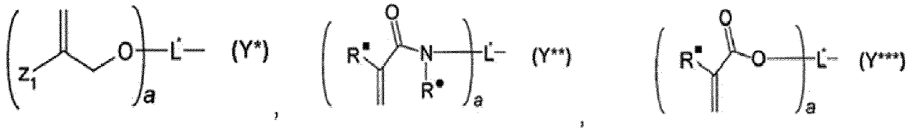 wherein
Z1 is COORα, COSRβ, CON(Rα)2, CONRαRβ, or CONHRα, wherein Rα and Rβ independently represent a hydrogen atom, a C1-18 alkyl group optionally substituted by a C3-8 cycloalkyl group, an optionally substituted C3-8 cycloalkyl group, an optionally substituted C4-18 aryl or heteroaryl group, an optionally substituted C5-18 alkylaryl or alkylheteroaryl group, or an optionally substituted C7-30 aralkyl group, whereby one Rα and one Rβ residue may form together with the adjacent nitrogen atom to which they are bound a 5- to 7-membered heterocyclic ring which may contain further nitrogen atoms or an oxygen atoms, and whereby the optionally substituted groups may be substituted by 1 to 5 C1-5 alkyl group(s);
R■ and R● independently represent a hydrogen atom, an optionally substituted C1-18 alkyl group, an optionally substituted C3-18 cycloalkyl group, an optionally substituted C5-18 aryl or heteroaryl group, an optionally substituted C5-18 alkylaryl or alkylheteroaryl group, an optionally substituted C7-30 aralkyl group, whereby the optionally substituted groups may be substituted by 1 to 5 C1-5 alkyl group(s);
L* represents an (a+b)-valent organic residue (whereby b is 1 when Y in Formula (A) is within the round brackets) containing 2 to 45 carbon atoms and optionally heteroatoms such as oxygen, nitrogen and sulfur atoms, the carbon atoms including a + b carbon atoms selected from primary and secondary aliphatic carbon atoms, secondary alicyclic carbon atoms, and aromatic carbon atoms, each of the a+b carbon atoms linking a phosphate or a moiety of any one of formula (Y*), (Y**) and (Y***); a is an integer of from 1 to 10, preferably 1 to 5; b is an integer of from 1 to 10, preferably 1 to 5; provided that at least one Y is not hydrogen. The preparation of such compounds wherein Y = Y* is known from EP 1 548 021 A1, the disclosure of which is herein incorporated by reference in its entirety.

Furthermore, one or more polymerizable monomers having at least one ethylenically unsaturated group may also be selected from phosphonic acid group containing polymerizable acidic compounds of the following formula (B):

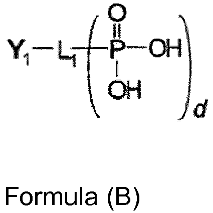

Wherein the moiety Y1 represents a moiety of the following formulae (Y1**) or (Y1***): 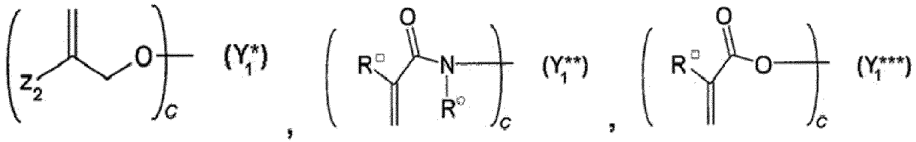
Z2 independently has the same meaning as defined for Z1;
R□ and R○ independently have the same meaning as defined for R■ and R●;
L1 represents a (c + d) valent organic residue containing 2 to 45 carbon atoms and optionally heteroatoms such as oxygen, nitrogen and sulfur, the carbon atoms including c + d carbon atoms selected from primary and secondary aliphatic carbon atoms, secondary alicyclic carbon atoms, and aromatic carbon atoms, each of the c+d carbon atoms linking a phosphonate or a moiety of any one of formula (Y1*), (Y1**) and (Y1***); and
c and d independently represent integers of from 1 to 10.

From the phosphoric acid ester group containing polymerizable compound having at least one polymerizable double bond, compounds of Formula (A') characterized by one of the following formulae are particularly preferred: 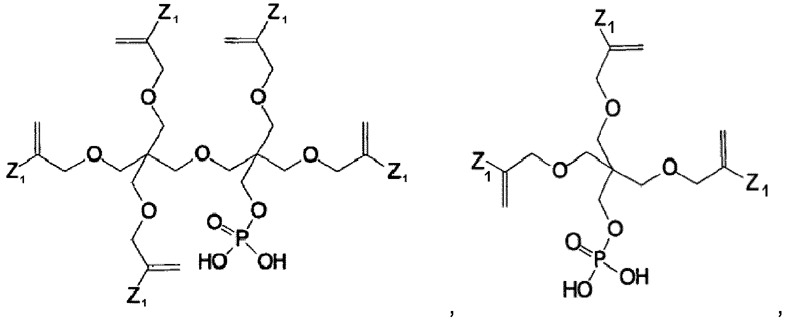 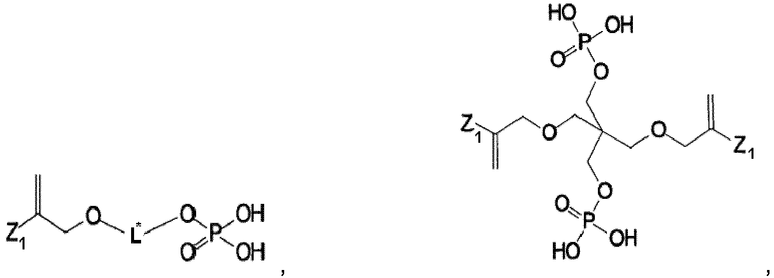 wherein Z1 is defined as above, and L* is an optionally substituted alkylene group. More preferably, Z1 is methyl, and L* is a C4 to C16 alkylene group. Even more preferably, L* is a C8 to C12 alkylene group.

### A radical initiator system

The dental composition of the present disclosure includes a radical initiator system. The radical initiator system may include a photoinitiator. Suitable photoinitiators may be in the form of a binary or tertiary system. A binary system may include a photosensitizer and a CH-acidic compound as coinitiator. A tertiary system may further include additionally an iodonium, sulfonium or phosphonium salt.

In the context of the present invention, the expression "CH-acidic compound" refers to an "a-CH-acidic compound". This definition at the latest is clear understandable (s.a. general textbooks of organic chemistry like "Organikum").

Such CH-acidic compounds can comprise a further functionality, such as at least one ethylenically unsaturated group, which allows taking part in the polymerization process as the at least one polymerizable monomer.

This can be an additional advantage of making use such specific coinitiators, which are not only suitable to replace toxic amine based coinitiators, but also offers the possibility of being incorporated into the growing polymerization chain.

In one embodiment, the at least one CH-acidic compound is a compound according to the following formula (I): 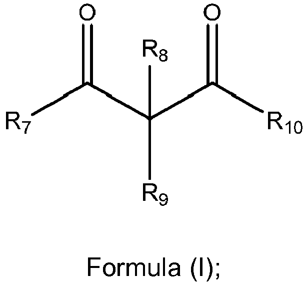 wherein
R_{7,} R₁₀ = a C₁ - C₈ alkoxy, a C₁ - C₈ alkyl, a hydroxy, a thiol, an amine, or a - OCH₂CH₂OC(O)C(CH₃)CH₂ moiety; preferably a methoxy, an ethoxy, a hydroxy, an amine, a methyl, or a -OCH₂CH₂OC(O)C(CH₃)CH₂ moiety; and
R_{8,} R₉ = a hydrogen, a C₁ - C₈ alkyl, or an acetyl moiety; preferably a hydrogen, a methyl, or an acetyl moiety; with the proviso that at least R₈ or R₉ is a hydrogen moiety.

In a preferred embodiment thereof, the at least one CH-acidic compound is a compound according to one of the following formulas (Ia) and (Ib): 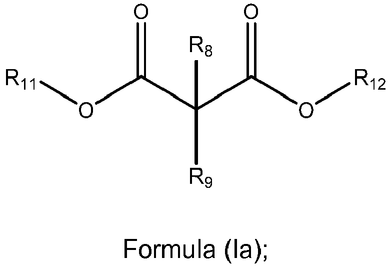
wherein R_{8,} R₉ = a hydrogen, a C₁ - C₈ alkyl, or an acetyl moiety; preferably a hydrogen, a methyl, or an acetyl moiety; with the proviso that at least R₈ or R₉ is a hydrogen moiety; and
R_{11,} R₁₂ = a hydrogen, a C₁ - C₈ alkyl, or a -CH₂CH₂OC(O)C(CH₃)CH₂ moiety; preferably a hydrogen, a methyl, an ethyl or a -CH₂CH₂OC(O)C(CH₃)CH₂ moiety; 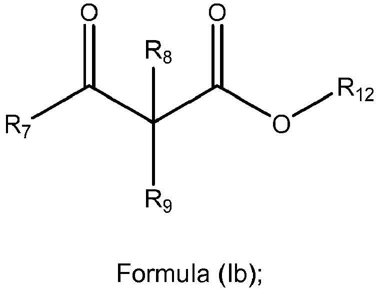
wherein
R₇ = a C₁ - C₈ alkoxy, a C₁ - C₈ alkyl, a hydroxy, a thiol, an amine, or a - OCH₂CH₂OC(O)C(CH₃)CH₂ moiety; preferably a methoxy, an ethoxy, a hydroxy, an amine, a methyl, or a -OCH₂CH₂OC(O)C(CH₃)CH₂ moiety;
R_{8,} R₉ = a hydrogen, a C₁ - C₈ alkyl, or an acetyl moiety; preferably a hydrogen, a methyl, or an acetyl moiety; with the proviso that at least R₈ or R₉ is a hydrogen moiety; and
R₁₂ = a hydrogen, a C₁ - C₈ alkyl, or a -CH₂CH₂OC(O)C(CH₃)CH₂ moiety; preferably a hydrogen, a methyl, an ethyl or a -CH₂CH₂OC(O)C(CH₃)CH₂ moiety;
preferably wherein the at least one CH-acidic compound is ethyl 2-methylacetoacetate (HD-3), ethyl diacetoacetate (HD-5), or 2-(methacryloyloxy) ethyl acetoacetate (HD-10).

In one alternative embodiment, the at least one CH-acidic compound is a compound according to the following formula (II): 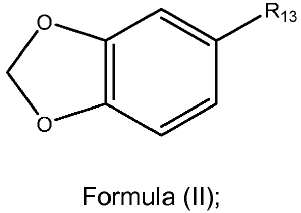 wherein
R₁₃ = a hydrogen, a C₁ - C₈ alkyl, a carboxylic acid, an acetyl, or a - CH₂OC(O)C(CH₃)CH₂ moiety; wherein the C₁ - C₈ alkyl moiety can be substituted by at least a nitrile group, a hydroxy group, or a carboxylic acid group;
preferably a methyl, a -CH₂OH, a -COOH, a -CH₂COOH, a -CH₂CN, an acetyl, or a -CH₂OC(O)C(CH₃)CH₂ moiety.

In a preferred embodiment thereof, the at least one CH-acidic compound is a compound according to one of the following formulas (IIa) and (IIb): 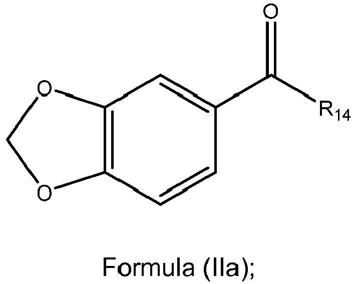 wherein
R₁₄ = a hydrogen, a C₁ - C₈ alkyl, or a hydroxy moiety; preferably a methyl or a hydroxy moiety. 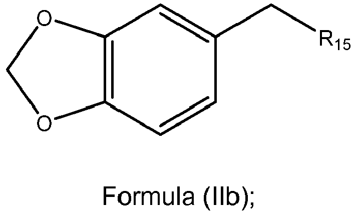 wherein
R₁₅ = a hydrogen, a hydroxy, a carboxylic acid, a nitrile, or a -OC(O)C(CH₃)CH₂ moiety.

In another alternative embodiment, the at least one CH-acidic compound is a compound according to the following formula (III): 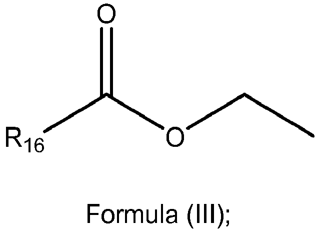 wherein
R₁₆ = a hydrogen, a C₁ - C₁₈ alkyl, or a C₁ - C₁₈ cycloalkyl moiety; wherein in the C₁ - C₈ alkyl or cycloalkyl moiety at least one carbon atom can be substituted by nitrogen; and wherein the C₁ - C₈ alkyl or cycloalkyl moiety can be substituted by at least a double bonded oxygen atom, an aromatic and/or an heteroaromatic group;
preferably wherein the at least one CH-acidic compound is N-(diphenylmethylene) glycine ethyl ester (HD-1), ethyl 2-oxocyclopentanecarboxylate (HD-2), or ethyl 2-ethyl-2-methylacetoacetate (HD-4).

In another alternative embodiment, the at least one CH-acidic compound is a compound according to the following formula (IV): 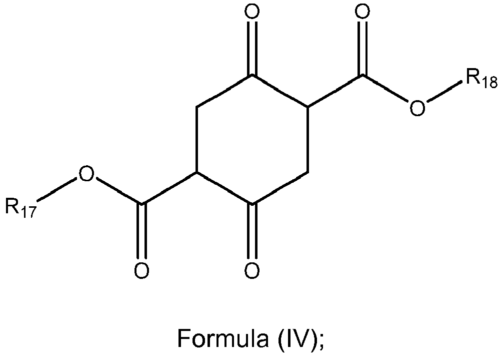 wherein
R₁₇, R₁₈ = a hydrogen or a C₁ - C₈ alkyl moiety;
preferably wherein the at least one CH-acidic compound is dimethyl 1,4-cyclohexanedione-2,5-dicarboxylate (HD-6).

In another alternative embodiment, the at least one CH-acidic compound is a compound according to the following formula (V): 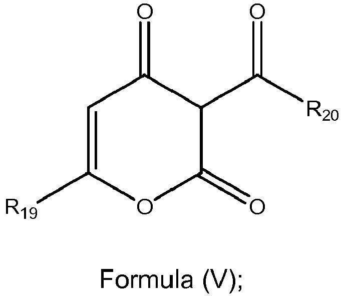 wherein
R_{19,} R₂₀ = a hydrogen or a C₁ - C₈ alkyl moiety;
preferably wherein the at least one CH-acidic compound is dehydroacetic acid (HD-7).

In another alternative embodiment, the at least one CH-acidic compound is a compound according to the following formula (VI): 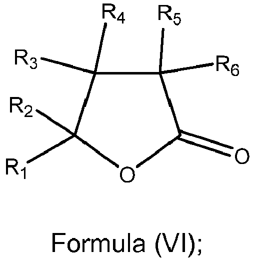 wherein
R_{1,} R_{2,} R_{3,} R_{4,} R_{5,} R₆ = a hydrogen, a C₁ - C₈ alkyl, a carboxylic acid, an acetyl, or a -OC(O)CHCH₂ moiety; with the proviso that at least R_{1,} R_{2,} R_{5,} or R_{6,} preferably R₅ or R_{6,} is a hydrogen moiety;
preferably wherein the at least one CH-acidic compound is a compound according to the following formula (VIa): 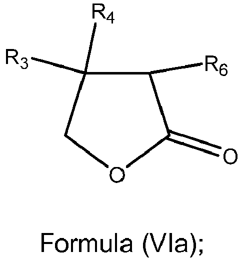
wherein
R_{3,} R_{4,} R₆ = a hydrogen, a C₁ - C₈ alkyl, a carboxylic acid, an acetyl, or a - OC(O)CHCH₂ moiety;
and more preferably wherein the at least one CH-acidic compound is α-acetyl-butyrolactone (HD-8) or (R)-α-acryloyloxy-β,β-dimethyl-γ-butyrolactone (HD-9).

In formula (VIa), R_{1,} R_{2,} and R₅ are a hydrogen moiety, respectively.

Conclusively, according to one embodiment of the present invention, the at least one CH-acidic compound is a compound according to one of the above-defined formulas (I), (Ia), (Ib), (II), (IIa), (IIb), (III), (IV), (V), (VI), and (Via).

If more than one CH-acidic compound is comprised by the inventive radical initiator system, all comprised CH-acidic compounds can be compounds according to the same formula (I), (Ia), (Ib), (II), (IIa), (IIb), (III), (IV), (V), (VI), or (Via).

Alternatively, if more than one CH-acidic compound is comprised by the inventive radical initiator system, each of the comprised CH-acidic compounds can be a compound according to a formula (I), (Ia), (Ib), (II), (IIa), (IIb), (III), (IV), (V), (VI), or (Via), wherein two CH-acidic compounds are never being compounds according to the same formula (I), (Ia), (Ib), (II), (IIa), (IIb), (III), (IV), (V), (VI), or (Via).

As a further alternative, if more than one CH-acidic compound is comprised by the inventive radical initiator system, at least two of the comprised CH-acidic compounds can be a compound according to the same formula (I), (Ia), (Ib), (II), (IIa), (IIb), (III), (IV), (V), (VI), or (VIa).

In one embodiment, the dental composition further comprises an additive selected from iodonium salts, phosphonium salts, and sulfonium salts; preferably wherein said additive is an iodonium salt.

In one preferred embodiment thereof, the additive is present in an amount of from 0.01 to 5 percent by weight based on the total weight of the dental composition.

Such an iodonium compound can comprise the following Formula (C):

R₂₁-I⁺-R₂₂ A⁻ Formula (C)

wherein
R₂₁ and R₂₂ are independent from each other, represent an organic moiety, and
A⁻ is an anion.

For example, the iodonium compound may be selected from (4-methylphenyl)[4-(2-methylpropyl) phenyl] iodonium hexafluoroantimonate, (4-methylphenyl)[4-(2-methylpropyl) phenyl] iodonium tetrafluoroborate, diphenyliodonium (DPI) tetrafluoroborate, di(4-methylphenyl)iodonium (Me2-DPI) tetrafluoroborate, phenyl-4-methylphenyliodonium tetrafluoroborate, di-(4-t-butylphenyl)-iodonium hexafluorophosphate, Bis(4-*tert*-butylphenyl)iodonium p-toluenesulfonate, di(4-heptylphenyl)iodonium tetrafluoroborate, di(3-nitrophenyl)iodonium hexafluorophosphate, di(4-chlorophenyl)iodonium hexafluorophosphate, di(naphthyl)iodonium tetrafluoroborate, di(4-trifluoromethylphenyl)iodonium tetrafluoroborate, DPI hexafluorophosphate, Me2-DPI hexafluorophosphate, DPI hexafluoroarsenate, di(4-phenoxyphenyl)iodonium tetrafluoroborate, phenyl-2-thienyliodonium hexafluorophosphate, 3,5-dimethylpyrazolyl-4-phenyliodonium hexafluorophosphate, DPI hexafluoroantimonate, 2,2'-DPI tetrafluoroborate, di(2,4-dichlorophenyl)iodonium hexafluorophosphate, di(4-bromophenyl)iodonium hexafluorophosphate, di(4-methoxyphenyl)iodonium hexafluorophosphate, di(3-carboxyphenyl)iodonium hexafluorophosphate, di(3-methoxycarbonylphenyl)iodonium hexafluorophosphate, di(3-methoxysulfonylphenyl)iodonium hexafluorophosphate, di(4-acetamidophenyl)iodonium hexafluorophosphate, di(2-benzothienyl)iodonium hexafluorophosphate, and DPI hexafluorophosphate.

In embodiments, iodonium compounds include diphenyliodonium (DPI) hexafluorophosphate, di(4-methylphenyl)iodonium (Me2-DPI) hexafluorophosphate, di-(4-t-butylphenyl)-iodonium hexafluorophosphate , diaryliodonium hexafluoroantimonate, (4-methylphenyl)[4-(2-methylpropyl) phenyl] iodonium hexafluoroantimonate, (4-methylphenyl)[4-(2-methylpropyl)phenyl]iodonium hexafluorophosphate (Irgacure® 250, commercial product available from BASF SE), (4-methylphenyl)[4-(2-methylpropyl) phenyl] iodonium tetrafluoroborate, 4-octyloxyphenyl phenyliodonium hexafluoroantimonate, 4-(2-hydroxytetradecyloxyphenyl)phenyliodonium hexafluoroantimonate, and 4-isopropyl-4'-methyldiphenyliodonium borate.

According to a further embodiments, the iodonium compound is di-(4-t-butylphenyl)-iodonium hexafluorophosphate, di(4-methylphenyl)iodonium (Me2-DPI) hexafluorophosphate, or Bis(4-*tert*-butylphenyl)iodonium *p*-toluenesulfonate.

According to yet further embodiments, the photo initiator may include a sulfonium compound of the following Formula (D):

R₂₃ R₂₄ R₂₅ S⁺ A⁻ Formula (D)

wherein
R₂₃, R₂₄ and R₂₅ are independent from each other, represent an organic moiety or wherein any two of R₂₃, R₂₄ and R₂₅ form a cyclic structure together with the sulfur atom to which they are bound, and
A⁻ is an anion.

The triaryl sulfonium salt may be S-(phenyl)thianthrenium hexafluorophosphate:

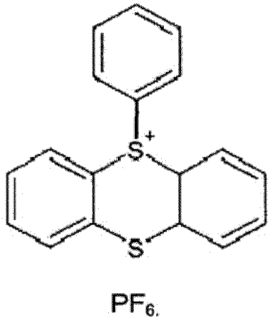

According to a further embodiment, the photo initiator may include a phosphonium compound of the following Formula (E):

R₂₆ R₂₇ R₂₈ R₂₉ P⁺ A⁻ Formula (E)

wherein
R₂₆, R₂₇, R₂₈ and R₂₉ are independent from each other, represent an organic moiety, and
A⁻ is an anion.

The phosphonium salts of Formula (E) may be tetraalkyl phosphonium salts tetrakis-(hydroxymethyl)-phosphonium (THP) salt or a tetrakis-(hydroxymethyl)-phosphonium hydroxide (THPOH) salt, wherein the anion of the tetraalkyl phosphonium salt is selected from the group consisting of formate, acetate, phosphate, sulphate, fluoride, chloride, bromide and iodide.

Other suitable photoinitiators for polymerizing free radically photopolymerizable compositions may include the class of phosphine oxides that typically have a functional wavelength range of about 380 nm to about 1200 nm. Further suitable phosphine oxide free radical initiators with a functional wavelength range of about 380 nm to about 450 nm are acyl and bisacyl phosphine oxides.

Commercially available phosphine oxide photoinitiators capable of free-radical initiation when irradiated at wavelength ranges of greater than about 380 nm to about 450 nm may include 1-hydroxy cyclohexyl phenyl ketone (IRGACURE 184), 2,2-dimethoxy-1,2-diphenylethan-1-one (IRGACURE 651), bis(2,4,6-trimethylbenzoyl)phenylphosphineoxide (IRGACURE 819), 1-[4-(2-hydroxyethoxy)phenyl]-2-hydroxy-2-methyl-1-propane-1-one (IRGACURE 2959), 2-benzyl-2-dimethylamino-1-(4-morpholinophenyl)butanone (IRGACURE 369), 2-methyl-1-[4-(methylthio)phenyl]-2-morpholinopropan-1-one (IRGACURE 907), and 2-hydroxy-2-methyl-1-phenyl propan-1-one (DAROCUR 1173),bis(2,4,6-trimethylbenzoyl) phenyl phosphine oxide (IRGACURE 819), bis(2,6-dimethoxybenzoyl)-(2,4,4-trimethylpentyl) phosphine oxide (CGI 403), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700), a 1:1 mixture, by weight, of bis(2,4,6-trimethylbenzoyl)phenyl phosphine oxide and 2-hydroxy-2-methyl-1-phenylpropane-1-one (DAROCUR 4265), and ethyl 2,4,6-trimethylbenzylphenyl phosphinate (LUCIRIN LR8893X). Typically, the phosphine oxide is present in the composition in catalytically effective amounts, such as from 0.1 percent by weight to 5.0 percent by weight, based on the total weight of the composition.

In one embodiment, the photosensitizer is a 1, 2-diketone compound, preferably wherein the photosensitizer is present in an amount of from 0.01 to 5 percent by weight based on the total weight of the dental composition.

The alpha-diketone sensitizer compound may be selected from camphorquinone, 1,2-diphenylethane-1,2-dione (benzil), 1,2-cyclohexanedione, 2,3-pentanedione, 2,3-hexanedione, 3,4-hexanedione, 2,3-heptanedione, 3,4-heptanedione glyoxal, biacetyl, 3,3,6,6-tetramethylcyclohexanedione, 3,3,7,7-tetramethyl-1,2-cycloheptanedione, 3,3,8,8-tetramethyl-1,2-cyclooctanedione; 3,3,18,18-tetramethyl-1,2-cyclooctadecanedione; dipivaloyl; furil, hydroxybenzil, 2,3-butanedione, 2,3-octanedione, 4,5-octanedione, and 1-phenyl-1,2-propanedione.

In one embodiment, the radical initiator system is present in an amount of from 0.1 to 10 percent by weight based on the total weight of the dental composition.

In one embodiment, the at least one CH-acidic compound is present in an amount of from 0.01 to 5 percent by weight based on the total weight of the dental composition.

In one embodiment, the dental composition is substantially free, preferably completely free, of amines, especially of tertiary amines, more especially of tertiary aromatic amines.

The present invention thus addresses the problem of providing an inventive dental composition comprising an amended initiator system.

The following non-limiting examples are provided to illustrate an embodiment of the present invention and to facilitate understanding of the invention but are not intended to limit the scope of the invention, which is defined by the claims appended hereto.

Compounds used in inventive and comparative experiments are given in the following: 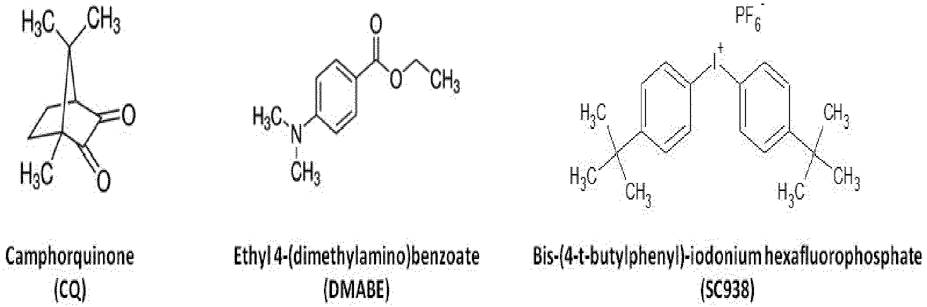 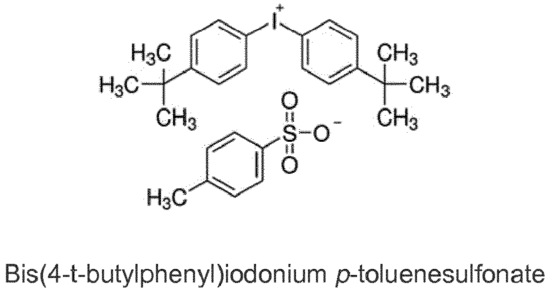 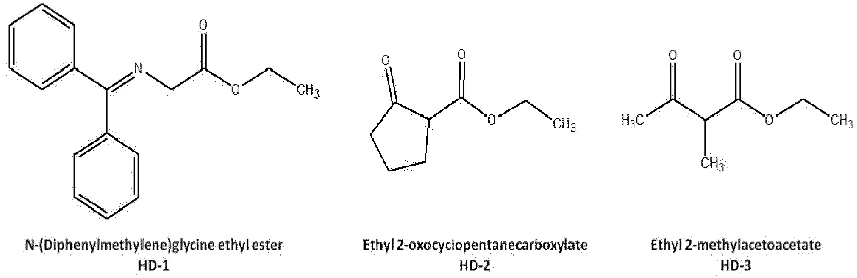 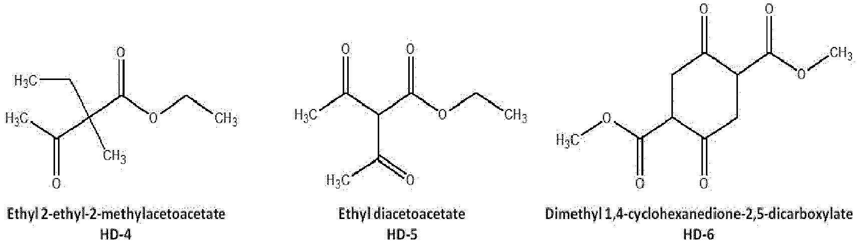 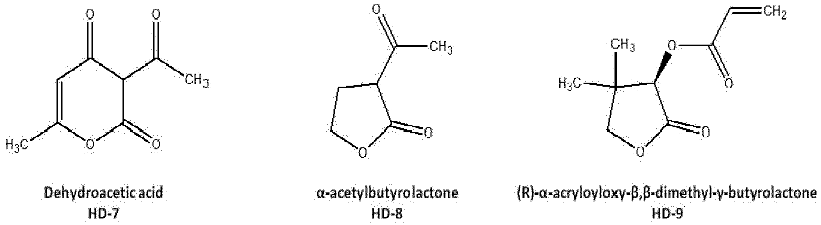 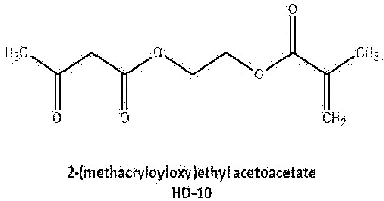 Herein, "SC938" refers to "speedcure 938".

### Example 1: Use of (R)-α-acyloyl-γ-butyrolactone (HD-8) as coinitiator for polymerization of TPH3 resin (a methacrylate resin)

Photopolymerization profiles of methacrylate functions (TPH3) in presence of (1) CQ (0.5% wt) or (2) CQ/lactone (HD-8) (0.5/3% w/w) or (4) CQ/ DMABE (0.5/1 % w/w) or (3) CQ/lactone (HD-8)/Speedcure 938 (0.5/3/1% w/w) under exposure to Smartlite Focus (300 mW.cm⁻²); sample thickness 1.4mm; under air. (as shown in **FIG.1**). It was observed that there is good reactivity of CQ/lactone (HD-8)/speedcure 938 in TPH3. It is similar to CQ/ DMABE, but amine free system.

Herein, it is clearly demonstrated that the use of a CH-acidic coinitiator in combination with CQ is better performing than CQ alone. Furthermore, it is shown that CQ with the CH-acidic coinitiator and an iodonium compound in a ternary system is even still much better performing than the binary system of CQ with the respective identical CH-acidic coinitiator. The comparative example of CQ with the tertiary amine DMABE shows a similar performance than the inventive ternary system example. This proves that the present invention has successfully provided a suitable dental composition comprising an initiator system, which can deliver the same performance than the environmentally toxic commonly used initiator system of CQ in combination with a tertiary amine as coinitiator.

### Example 2: Use of (R)-α-acyloyl-γ-butyrolactone (HD-8) as coinitiator for polymerization of Prime & bond Active resin (a methacrylate resin)

Photopolymerization profiles of C=C double bonds (PrimeBond Active) in presence of (1) CQ (1.5% w/w) or (2) CQ/Speedcure 938 (1.5/0.75 % w/w) or (3) CQ/acetylbutyrolactone (HD-8)/ Bis(4-t-butylphenyl)iodonium p-toluenesulfonate (1.5/1.2/0.75 % w/w) or (4) CQ/acetylbutyrolactone (HD-8)/Speedcure938 (1.5/1.2/0.75 % w/w) or under exposure to SmartLite Focus (300mW.cm-2); sample thickness 17µm; under air **(****Figure 2****).**

It was observed that there is good reactivity of CQ/lactone(HD-8)/speedcure 938 and CQ/lactone/iodoinium sulfonate in PBA. It is an amine free system. Slight tacky polymers were obtained.

Herein, it is clearly demonstrated that the performance of a ternary inventive initiator system of CQ in combination with a CH-acidic compound and an iodonium compound is better than either CQ alone or the combination of CQ with an iodonium ion. This proves that the suitable performance shown in Figure 1 is depending on the CH-acidic compound. A combination of an iodonium compound with CQ is not superior and does not represent an alternative. However, if additionally used with CQ and a CH-acidic compound, the use of such an iodonium compound has shown to be senseful in order to further improve the performance of the binary system of CQ and the respective CH-acidic compound.

### Example 3. Systematic investigation of a plurality of CH-acidic compounds in combination with CQ plus comparison to CQ alone and in combination with a tertiary amine (DMABE)

Photopolymerization profiles of methacrylate functions (TPH3) in presence of camphorquinone as photoinitiator (under air; thickness = 1.4 mm; SmartLite Focus 300 mW.cm⁻²): (1) CQ/HD-1/SC938 (0.5/3/1 % w/w) (2) CQ/HD-2/SC938 (0.5/3/1 % w/w) (3) CQ/HD-3/SC938 (0.5/3/1 % w/w) (4) CQ/HD-4/SC938 (0.5/3/1 % w/w) (5) CQ/HD-5/SC938 (0.5/3/1 % w/w) (6) CQ/HD-6/SC938 (0.5/3/1 % w/w) (7) CQ/HD-7/SC938 (0.5/3/1 % w/w) (8) CQ/HD-8/SC938 (0.5/3/1 % w/w) (9) CQ/HD-10/SC938 (0.5/3/1 % w/w) (10) CQ/DMABE (0.5/0.5 % w/w) (11) CQ (0.5% w/w). The irradiation starts for t = 5 s (as depicted in FIG. 3).

New CH-acidic compounds (HD-1 to HD-10) were used as efficient coinitiators in combination with camphorquinone (CQ) for the free radical polymerization of methacrylates under air and under exposure to a blue dental LED (SmartLite Focus - Dentsply Sirona). Remarkably, they can be used in combination with CQ and an iodonium salt and these amine-free systems present excellent polymerization performances, even in comparison to the current comparative reference systems CQ/DMABE and CQ alone. For example, the polymerizations of TPH3 resin (a methacrylate resin) in presence of CQ/CH-acidic compound/SC938 using 9 different CH-acidic compounds as coinitiator in the respectively investigated photoinitiating system are shown in **Figure 3****.**

Herein, it is again proven that the performance of a ternary inventive initiator system of CQ in combination with a CH-acidic compound and an iodonium compound is better than either CQ alone or at least similar to a combination of CQ with a commonly used tertiary amine (DMABE) as coinitiator.

### Example 4. Use of (R)-α-acryloyloxy-β,β-dimethyl-γ-butyrolactone (HD-9) as coinitiator

(R)-α-acryloyloxy-β,β-dimethyl-γ-butyrolactone (HD-9) was proposed as a new coinitiator in combination with camphorquinone. For example, the polymerizations of TPH3 resin in presence of CQ/HD-9, CQ/HD-9/SC938 and CQ/DMABE/HD-9 as photoinitiating systems are shown in **FIG. 4****.** The amine-free system CQ/HD-9/SC938 presents excellent polymerization performances similar to the reference comparative system CQ/DMABE.

Photopolymerization profiles of methacrylate functions (TPH3) in presence of camphorquinone as photoinitiator (under air; thickness = 1.4 mm; SmartLite Focus 300 mW.cm⁻²): (1) CQ/TPH3 (0.5/100 % w/w) (2) CQ/HD-9/TPH3 (0.5/5/95 % w/w) (3) CQ/HD-9/SC938/TPH3 (0.5/5/1/100 % w/w) (4) CQ/DMABE/TPH3 (0.5/0.6/100 % w/w). The irradiation starts for t = 5 s. (as depicted in **FIG. 4**).

While the principles of the invention have been explained in relation to certain embodiments, it is to be understood that various modifications thereof will become apparent to those skilled in the art upon reading the specification. Therefore, it is to be understood that the invention disclosed herein is intended to cover such modifications as fall within the scope of the appended claims. The scope of the invention is limited only by the scope of the appended claims.

## Claims

1. Dental composition comprising
c) at least one polymerizable monomer having at least one ethylenically unsaturated group;
d) a radical initiator system comprising
iii. a photosensitizer having an absorption maximum ranging from 400 nm to 800 nm; and
iv. at least one CH- acidic compound as coinitiator.

2. Dental composition according to claim 1 **characterized in that** the at least one CH-acidic compound is a compound according to the following formula (I): 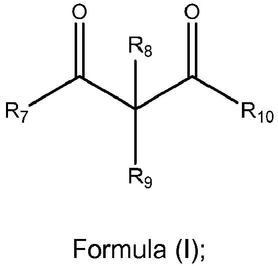 wherein
R₇, R₁₀ = a C₁ - C₈ alkoxy, a C₁ - C₈ alkyl, a hydroxy, a thiol, an amine, or a -OCH₂CH₂OC(O)C(CH₃)CH₂ moiety; preferably a methoxy, an ethoxy, a hydroxy, an amine, a methyl, or a -OCH₂CH₂OC(O)C(CH₃)CH₂ moiety; and
R₈, R₉ = a hydrogen, a C₁ - C₈ alkyl, or an acetyl moiety; preferably a hydrogen, a methyl, or an acetyl moiety; with the proviso that at least R₈ or R₉ is a hydrogen moiety.

3. Dental composition according to claim 2 **characterized in that** the at least one CH-acidic compound is a compound according to one of the following formulas (Ia) and (Ib): 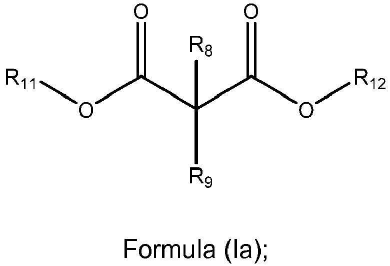 wherein
R₈, R₉ = a hydrogen, a C₁ - C₈ alkyl, or an acetyl moiety; preferably a hydrogen, a methyl, or an acetyl moiety; with the proviso that at least R₈ or R₉ is a hydrogen moiety; and
R₁₁, R₁₂ = a hydrogen, a C₁ - C₈ alkyl, or a -CH₂CH₂OC(O)C(CH₃)CH₂ moiety; preferably a hydrogen, a methyl, an ethyl or a - CH₂CH₂OC(O)C(CH₃)CH₂ moiety; 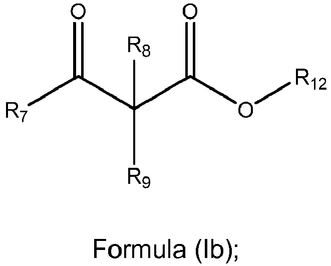
wherein
R₇ = a C₁ - C₈ alkoxy, a C₁ - C₈ alkyl, a hydroxy, a thiol, an amine, or a - OCH₂CH₂OC(O)C(CH₃)CH₂ moiety; preferably a methoxy, an ethoxy, a hydroxy, an amine, a methyl, or a -OCH₂CH₂OC(O)C(CH₃)CH₂ moiety;
R_{8,} R₉ = a hydrogen, a C₁ - C₈ alkyl, or an acetyl moiety; preferably a hydrogen, a methyl, or an acetyl moiety; with the proviso that at least R₈ or R₉ is a hydrogen moiety; and
R₁₂ = a hydrogen, a C₁ - C₈ alkyl, or a -CH₂CH₂OC(O)C(CH₃)CH₂ moiety; preferably a hydrogen, a methyl, an ethyl or a -CH₂CH₂OC(O)C(CH₃)CH₂ moiety;
preferably wherein the at least one CH-acidic compound is ethyl 2-methylacetoacetate (HD-3), ethyl diacetoacetate (HD-5), or 2-(methacry-loyloxy)ethyl acetoacetate (HD-10).

4. Dental composition according to claim 1 **characterized in that** the at least one CH-acidic compound is a compound according to the following formula (II): 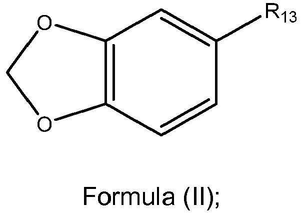 wherein
R₁₃ = a hydrogen, a C₁ - C₈ alkyl, a carboxylic acid, an acetyl, or a - CH₂OC(O)C(CH₃)CH₂ moiety; wherein the C₁ - C₈ alkyl moiety can be substituted by at least a nitrile group, a hydroxy group, or a carboxylic acid group;
preferably a methyl, a -CH₂OH, a -COOH, a -CH₂COOH, a -CH₂CN, an acetyl, or a -CH₂OC(O)C(CH₃)CH₂ moiety.

5. Dental composition according to claim 4 **characterized in that** the at least one CH-acidic compound is a compound according to one of the following formulas (IIa) and (IIb): 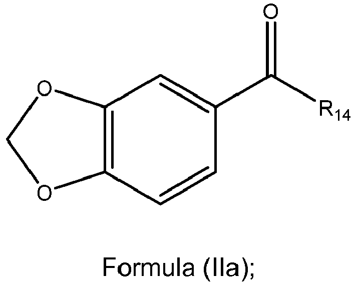 wherein
R₁₄ = a hydrogen, a C₁ - C₈ alkyl, or a hydroxy moiety; preferably a methyl or a hydroxy moiety. 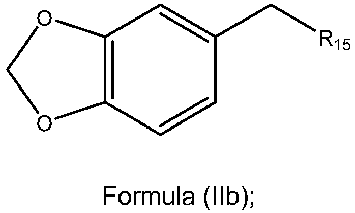 wherein
R₁₅ = a hydrogen, a hydroxy, a carboxylic acid, a nitrile, or a - OC(O)C(CH₃)CH₂ moiety.

6. Dental composition according to claim 1 **characterized in that** the at least one CH-acidic compound is a compound according to the following formula (III): 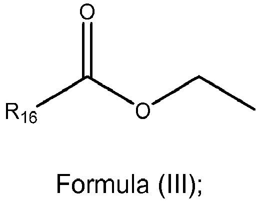 wherein
R₁₆ = a hydrogen, a C₁ - C₁₈ alkyl, or a C₁ - C₁₈ cycloalkyl moiety; wherein in the C₁ - C₈ alkyl or cycloalkyl moiety at least one carbon atom can be substituted by nitrogen; and wherein the C₁ - C₈ alkyl or cycloalkyl moiety can be substituted by at least a double bonded oxygen atom, an aromatic and/or an heteroaromatic group;
preferably wherein the at least one CH-acidic compound is N-(diphenylmethylene) glycine ethyl ester (HD-1), ethyl 2-oxocyclopentanecarboxylate (HD-2), or ethyl 2-ethyl-2-methylacetoacetate (HD-4).

7. Dental composition according to claim 1 **characterized in that** the at least one CH-acidic compound is a compound according to the following formula (IV): 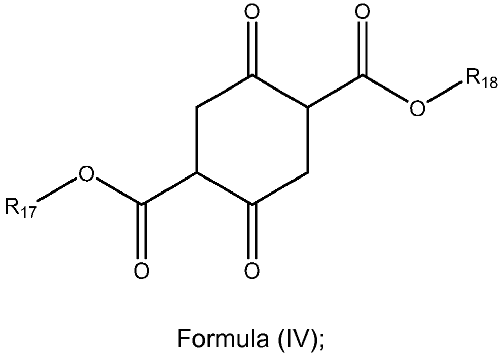 wherein
R_{17,} R₁₈ = a hydrogen or a C₁ - C₈ alkyl moiety;
preferably wherein the at least one CH-acidic compound is dimethyl 1,4-cyclohexanedione-2,5-dicarboxylate (HD-6).

8. Dental composition according to claim 1 **characterized in that** the at least one CH-acidic compound is a compound according to the following formula (V): 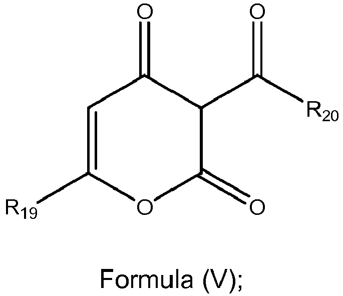 wherein
R_{19,} R₂₀ = a hydrogen or a C₁ - C₈ alkyl moiety;
preferably wherein the at least one CH-acidic compound is dehydroacetic acid (HD-7).

9. Dental composition according to claim 1 **characterized in that** the at least one CH-acidic compound is a compound according to the following formula (VI): 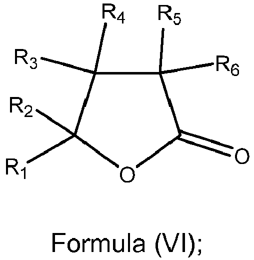 wherein
R_{1,} R_{2,} R_{3,} R_{4,} R_{8,} R₆ = a hydrogen, a C₁ - C₈ alkyl, a carboxylic acid, an acetyl, or a -OC(O)CHCH₂ moiety; with the proviso that at least R_{1,} R_{2,} R_{8,} or R₆, preferably R₅ or R_{6,} is a hydrogen moiety;
preferably wherein the at least one CH-acidic compound is a compound according to the following formula (VIa): 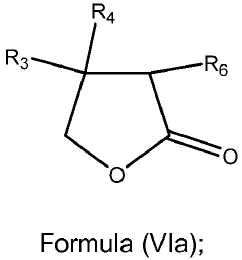
wherein
R_{3,} R_{4,} R₆ = a hydrogen, a C₁ - C₈ alkyl, a carboxylic acid, an acetyl, or a - OC(O)CHCH₂ moiety;
and more preferably wherein the at least one CH-acidic compound is α-acetylbutyrolactone (HD-8) or (R)- α-acryloyloxy-β,β-dimethyl-γ-butyrolactone (HD-9).

10. Dental composition according to one of the preceding claims **characterized in that** the dental composition further comprises an additive selected from iodonium salts, phosphonium salts, and sulfonium salts; preferably wherein said additive is an iodonium salt.

11. Dental composition according to claim 10 **characterized in that** the additive is present in an amount of from 0.01 to 5 percent by weight based on the total weight of the dental composition.

12. Dental composition according to one of the preceding claims **characterized in that** the photosensitizer is a 1, 2-diketone compound, preferably wherein the photosensitizer is present in an amount of from 0.01 to 5 percent by weight based on the total weight of the dental composition.

13. Dental composition according to one of the preceding claims **characterized in that** the radical initiator system is present in an amount of from 0.1 to 10 percent by weight based on the total weight of the dental composition.

14. Dental composition according to one of the preceding claims **characterized in that** the at least one CH-acidic compound is present in an amount of from 0.01 to 5 percent by weight based on the total weight of the dental composition.

15. Dental composition according to one of the preceding claims **characterized in that** the dental composition is substantially free, preferably completely free, of amines, especially of tertiary amines, more especially of tertiary aromatic amines.
